Europäisches Patentamt

European Patent Office

Office européen des brevets

(11) Publication number: 0 369 458 A2

# EUROPEAN PATENT APPLICATION

(21) Application number: 89121295.3

(51) Int. Cl.5: **C12N 15/85, C12N 15/16, C12N 15/67**

(22) Date of filing: 17.11.89

(30) Priority: 18.11.88 US 274241

(43) Date of publication of application:
23.05.90 Bulletin 90/21

(84) Designated Contracting States:
AT BE CH DE ES FR GB GR IT LI LU NL SE

(71) Applicant: PHILLIPS PETROLEUM COMPANY
5th and Keeler
Bartlesville Oklahoma 74004(US)

(72) Inventor: Williams, Mark Edward
6919 Pear Tree Drive
Carlsbad, CA 92008(US)
Inventor: Murphy, Michael Francis
4505 Benhurst Avenue
San Diego, CA 92122(US)

(74) Representative: Dost, Wolfgang,
Dr.rer.nat.,Dipl.-Chem. et al
Patent- & Rechtsanwälte Bardehle .
Pagenberg . Dost . Altenburg . Frohwitter .
Geissler & Partner Galileiplatz 1 Postfach 86
06 20
D-8000 München 86(DE)

(54) Bovine metallothionein regulatory region.

(57) The present invention provides a bovine metallothionein II regulatory region and a modified bovine metallothionein regulatory region as well as vectors and methods for the utilization of the aforesaid regulatory regions.

FIG. 1

## BOVINE METALLOTHIONEIN REGULATORY REGION

This invention relates to the field of recombinant DNA biotechnology. In one of its aspects the invention relates to DNA fragments which regulate the transcription of DNA. In another aspect the invention relates to vectors which incorporate the above-described DNA fragment. In yet another aspect, the invention relates to novel cells transformed with the above-described vectors.

The present invention relates generally to the manipulation of genetic material, particularly to the regulation of the frequency of transcription of RNA from DNA. This invention specifically relates to DNA fragments containing the regulatory region of the bovine metallothionein II gene.

## Background of the Invention

As recombinant DNA biotechnology has developed in recent years, the controlled production by cells of an enormous variety of useful polypeptides has become possible. Many eukaryotic polypeptides, for example human growth hormone, leukocyte interferons, human insulin and human proinsulin have been produced by various recombinant cells. The continued application of techniques already in hand is expected in the future to permit recombinant production of a variety of other useful polypeptide products.

The basic techniques employed in the field of recombinant technology are known by those of skill in the art. The elements desirably present for the practice of recombinant DNA biotechnology include, but are not limited to:

(1) a gene encoding one or more desired polypeptide(s), operably linked (Operably linked refers to a juxtaposition wherein the components are configured so as to perform their usual function.) to a regulatory region which controls expression in the host cell;

(2) a vector, usually a plasmid into which a nucleotide sequence can be inserted; a vector is any nucelotide sequence-containing construct capable of transforming a host;

(3) a suitable host into which the desired nucleotide sequence can be transferred, where the host also has the cellular apparatus to express the information encoded in the transferred nucleotide sequence.

This invention discloses for the first time the bovine metallothionein II (bMT-II) regulatory region, a modified bovine metallothionein II regulatory region sequence (mbMT-II), vectors comprised of said regulatory regions, and mutants thereof. Mutants are understood to encompass base pair changes, additions, or deletions which do not significantly impair the function of the mutated nucleotide sequence. Additionally disclosed are methods of activating these bMT-II regulatory regions.

The bMT-II and mbMT-II regulatory regions regions are responsive to induction through the use of exogenous heavy metals. Regulatory response to an easily manipulatable exogenous environmental stimulus, such as the presence or absence of heavy metals, makes these regulatory regions of particular value and utility in recombinant DNA technology. A change in metal concentration will influence the expression of heterologous proteins. Furthermore, these regulatory regions are compatible with bovine cells and were derived from genes which encode metallothionein proteins.

These proteins are small, cysteine-rich proteins that bind unusually high amounts of heavy metals, such as Cd, Zn, Cu, Ag, and Hg. Specifically, the cysteine-rich areas of the proteins are believed to be directly involved in the binding of these metals. Metallothioneins are a widely distributed class of low molecular weight proteins present in most vertebrates and invertebrates. These proteins play an important role in cadmium and mercury detoxification, and also may be involved in zinc and copper homeostasis.

Numerous publications relate to metallothionein genes in other mammals, such as Stallings et al. "Assignment of Genes Encoding Metallothioneins I and II of Chinese Hamster Chromosome 3: Evidence for the Role of Chromosome Rearrangement in Gene Amplification", Molecular and Cellular Biology, Vol. 4, pp. 2932-2936 (1984); Durnam et al. "Isolation and Characterization of the Mouse Metallothionein-1 Gene", P.N.A.S., 77, pp. 6511-6515, (1980) Karin et al. "Characterization of DNA Sequences Through Which Cadmium and Glucocorticoid Hormones Induce Human Metallothionein-IIA Gene", Nature 308, pp. 513-519 (1984); Schmidt and Harner "Cloning and Sequencing Analysis of Two Monkey Metallothionein cDNA's", Gene, 24, 137-146 (1983). Three U.S. Patents have also issued in the area: Karin 4,601,978 (July 22, 1986), Palmiter et al. 4,579,821 (April 1, 1986) and Fogel et al. 4,511,652 (April 16, 1985). However, none of these publications elucidate the bovine metallothionein regulatory region.

Until the present invention no bovine metallothionein regulatory region had been identified. However, with the discovery of the bovine metallothionein II regulatory region, an effective bovine regulatory region has now been identified suitable for use in eukaryotic, including mammalian (particularly bovine) cells.

Thus an object of the invention is to provide a novel bovine metallothionein II (bMT-II) regulatory region responsive to the presence of heavy metals as well as vectors containing said bMT-II regulatory region. Another object of the invention is to provide a modified bovine metallothionein II (mbMT-II) regulatory region responsive to the presence of heavy metals, as well as vectors containing said mbMT-II regulatory region. Still another object of this invention are novel vectors comprising the bMT-I or mbMT-II regulatory region operably linked to heterologous nucleotide sequences. Yet another object of this invention are methods of activating the bMT and mbMT-II regulatory region to facilitate the expression of heterologous nucleotide sequences. Other aspects, objects and several advantages of this invention will be apparent from the foregoing specifications, examples and claims.

## Summary of the Invention

In accordance with the present invention, we have discovered, isolated and characterized a bovine DNA fragment consisting essentially of a bovine metallothionein regulatory region wherein said regulatory region is responsive to the presence of heavy metals within the host cell environment. The bMT-II regulatory region disclosed in this invention is characterized by being capable of controlling the transcription of DNA into RNA and inducible in the presence of heavy metals.

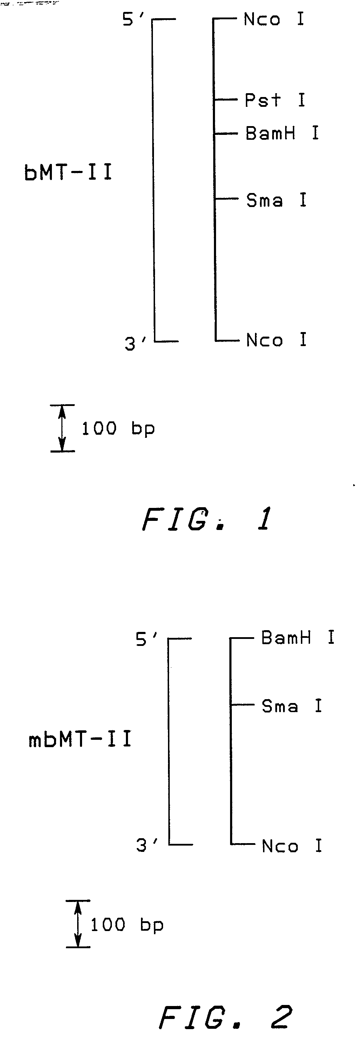

In another aspect of the present invention, we have also discovered, isolated, and characterized a modified bovine DNA fragment derived from the bMT-II regulatory region, which consists essentially of a DNA fragment wherein said DNA fragment is responsive to the presence of heavy metals within the host cell environment and has the restriction map of Figure 2. The mbMT-II regulatory region disclosed herein is characterized by being capable of controlling the transcription of DNA into RNA and inducible in the presence of heavy metals.

In a further aspect of this invention vectors incorporating the bMT-II or mbMT-II and methods for activating the bMT regulatory regions for the facilitation of the expression of heterologous genes are provided.

## Brief Description of the Figures

Figure 1 provides a restriction map of the bovine metallothionein II regulatory region.

Figure 2 provides a restriction map of the modified bovine metallothionein II regulatory region.

Figure 3 provides a restriction map of plasmid pL260. The PstI sites provided in this figure are provided in their approximate locations.

Figure 4 provides a restriction map of plasmid pL266. The PstI, BglII and ApaI sites in this figure are provided in their approximate locations.

Figure 5A provides a representational flow chart of the contruction of pL260.

Figure 5B provides a representational flow chart of the contruction of pL223.

Figure 5C provides a representational flow chart of the construction of pL264, pL265 and pL266.

In the attached Figures, restriction sites employed for manipulation of nucleotide sequences, but which are destroyed upon ligation are indicated by enclosing the abbreviation for the destroyed site in parentheses.

## Description of the Invention

In accordance with the present invention, there is provided a novel DNA fragment comprising a bovine regulatory region responsive to the presence of heavy metals within the host environment. A restriction map of the bMT-II regulatory region to provided in Figure 1. This regulatory region has been further characterized by the nucleotoide sequence which is provided in Table 1. The bMT-II regulatory region extends from about the Nco I site at approximately nucleotide -749 to about the Nco I site at approximately nucleotide -1 (as shown in Table 1). This nucleotide sequence includes approximately at least five apparent metal responsive elements. This regulatory region is capable of controlling the transcription of RNA when operably linked to and positioned at the 5′ end of a heterologous DNA sequence coding for a polypeptide.

3

EP 0 369 458 A2

## Table 1

```
                                                                      -676
CATGGCAGCTACAGTACAACATCAATAATCATCCCATGGATTCAGTAGGAGGCTACAAAAATTTTGAGTCTTC    ,
NcoI                              NcoI

                                                                      -603
ACAGGTACAAAAAGTTTTCTGTAATGTCCTGAGTTCCAACCTTTTTTTCCACCCGAGCTGGATGGCATCACTG

                                                                      -530
ATTCAATGGACATGAGTTTGAGTAAACTCTGGGAGTTGGTGATGGACAGGGAGGCCTGGAGTGCTGCAGTCCA
                                                                PstI

                                                                      -459
CGGGGTCGCAAAGGTCGGACACGACTGTGCGACTGAAATGAACTGAACTGAACTGAACAGGATCCT
                                                                  BamHI

                                                                      -368
CAGGATCAACTCTACTCAGGCCCGGCAAGCACAGGGTACAGAGCAGGGGGCAGGGGGAGGGGCGGGAGAGCTG

                                                                      -313
GGGGAGGGGCAGGCGGTGGCGGAAACTCTGCACACGCTGAGCGCAGGTGCCAGGGGCTCGGCGCACGGCCCGG
                             MRE 1     ===============    MRE 7
                                          MRE 6

                                                                      -241
GCCGCCCTGCACGCGGTGTTCCCCGCTCTGCGCCCGGTCCGCCCCTCCATCGCCCTGGCGCACGTAGGGCGG
     MR2                     MR3                       MRE 8

                                                                      -169
CTTCTGGGAACCGATGCCTGGCGGCCCGTGTGCACAGCTCGGTGAGCCGCGCGGCCCGCCAGTGACTCAGCG
                             MRE 4
                             ===============
                             MRE 9

                                                                      -96
CGGGGCGGGTTCAGGCAGCTCCCGGCCCCGGAGGGTTTTTGCACTCGTCCCAGCTCCTTTCCGCTATAAAGGC
                                         MRE 5

                                                                      -23
AGCCCACCGCCCGCTCCGCTCCAGCACGCCCCTTCGACACCCTCGCCATCCTTTGCTCAGCAGTCTCCGGACC

                    -1
CCAGCCTCCAGTTCAGCTCGCC
                    NcoI
```

              ———————— Metal Response Elements (MRE) in Sense Strand
              ======== Metal Response Elements (MRE) in anti-sense Strand
              ------- TATA Box
              -------

Also included within the scope of the invention are mutants of the above-described nucleotide sequence, said mutants having functional activity substantially the same as said DNA sequence.

Further in accordance with the present invention, there is provided a modified bovine metallothionein II regulatory region, (mbMT-II) wherein said regulatory region is responsive to the presence of heavy metals within the host environment. A restriction map of the mbMT-II regulatory region is provided in Figure 2. This regulatory region has been further characterized by the nucleotide sequence which is provided in Table 2. The mbMT-II regulatory region extends from about the BamHI site at approximately nucleotide -464 to about theNcoI site at approximately nucleotide -1 (as shown in Table 2). This sequence includes

4

approximately at least five apparent metal responsive elements as shown in Table 2. Also included within the scope of this invention are mutants of the mbMT-II nucleotide sequence wherein said mutants have functional activity substantially the same as said DNA sequence.

## Table 2

-391

GATCCTCAGGATCAACTCTACTCAGGCCCGGCAAGCACAGGGTACAGAGCAGGGGGCAGGGGGAGGGGCGGGAG
BamHI

-319

AGCTGGGGGAGGGGCAGGCGGTGGCGGAAACTCTGCACACGCTGAGCGCAGGTGCCAGGGGCTCGGCGCACG
                                    MRE 1              ==========  MRE 7
                                              MRE 6

-246

GCCCGGGCCGCCCTGCACGCGGTGTTCCCCGCTCTGCGCCCGGTCCGCCCCTCCATCGCCCTGGCGCACGTAG
        MRE 2                      MRE 3                    MRE 8

-172

GGCGGCTTCTGGGAACCGATGCCTGGCGGCCCGTGTGCACAGCTCGGTGAGCCGCGCGGCCCGCCAGTGACTCA
                            MRE 4
                            ============
                            MRE 9

-99

GCGCGGGGCGGGTTCAGGCAGCTCCCGGCCCCGGAGGGTTTTTGCACTCGTCCCAGCTCCTTTCCGCTATAAA
                                        MRE 5

-26

GGCAGCCCACCGCCCGCTCCGCTCCAGCACGCCCCTTCGACACCCTCGCCATCCTTTGCTCAGCAGTCTCCGG

-1

ACCCCAGCCTCCAGTTCAGCTCGCC
                    NcoI

_____ Metal Response Elements (MRE) in Sense Strand

===== Metal Response Elements (MRE) in anti-sense Strand

----- TATA Box

The bovine metallothionein regulatory region may be recovered, either from bovine cell lines such as MDBK (ATCC #CCL22) by methods analogous to those set forth in the following examples, or synthesized from the nucleotide sequences provided in Tables 1 or 2.

The general method for recovering a bovine regulatory region would consist of growing bovine cell lines in the presence of a heavy metal or metals for a prolonged period of time in incrementally higher concentrations of heavy metal. This exposure would result in high levels of metallothionein RNA being represent which could be recovered by an appropriate technique such as selecting poly A-containing molecules over oligo-T molecules. The RNA could then be recovered and used to synthesize cDNA which could be ligated into vectors to form a library. This would then be followed by probing the library with a short segment of a mammalian metallothionein gene to detect homologous regions by annealing. The cDNA which was isolated from the library and was homologous could then be used to probe the bovine genome in, for example, a Southern hybridization assay or a bovine genomic library to locate the bovine metallothionein gene and regulatory region.

This regulatory region could also be obtained by using the nucleotide sequence provided in Table 1 or 2 and synthesizing the sequence using enzymatic or chemical means. Suitable means include but are not limited to chemical procedures based on phosphotriester, phosphite, or cyanoethylphosphoramidite chemistry.

Those skilled in the art will also recognize that subsequently isolated regulatory regions may contain a de minimis number of nucleotide differences because of genetic drift or sequencing error which may have occurred.

Modification of the bovine regulatory region could also be performed by adding linker DNA to provide new restriction sites, removing the existing 5' or 3' restriction sites by blunting the ends of the fragment, or any other suitable means known to those skilled in the art.

Once the bovine metallothionein regulatory region is recovered it may be maintained or replicated in eukaryotic or prokaryotic plasmid-host system such as pBR322 maintained in E.coli or any other suitable system known in the art.

Those skilled in the art will recognize that numerous additional DNA sequences can also be incorporated into the vectors used in the practice of this invention, such as bacterial plasmid DNA, marker genes, bacteriophage DNA, autonomous replicating sequences or long terminal repeats to name only a few representative examples.

To utilize the bovine metallothionein regulatory regions disclosed herein the fragments described in Figure 1 and Figure 2 can be operably linked to heterologous DNA sequences. For the purpose of this specification heterologous DNA sequences are DNA sequences which do not naturally occur operably linked to the fragments described in Figures 1 and 2. Suitable heterologous DNA sequences encoding a polypeptide which could be operably linked with the bovine metallothionein regulatory region include but are not limited to those encoding the bovine growth hormone, human growth hormone, chicken growth hormone, and human growth hormone releasing factor. Heterologous DNA sequences used with the present invention should contain a 5' ATG start codon, a 3' stop codon and may additionally include nucleotide sequences which function to stabilize the mRNA, such as a sequence directing polyadenylation.

The combination of a bovine metallothionein regulatory region operably linked to a heterologous DNA sequence may be inserted in an appropriate vector for the chosen host cell. Numerous vector-host cell combinations are possible and are known to those skilled in the art. Vectors derived from suitably modified papovaviruses, adenoviruses, avian retroviruses and mammalian retroviruses are well suited for mammalian hosts. Additional sequences such as marker genes or other sequences which render the vector capable of growth amplification and rapid propagation in bacteria or yeast may also be present.

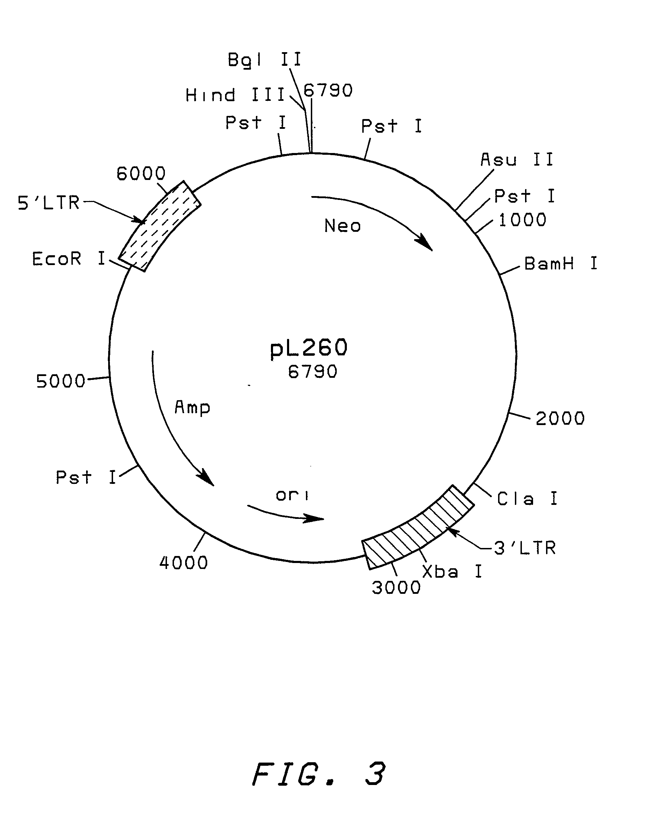

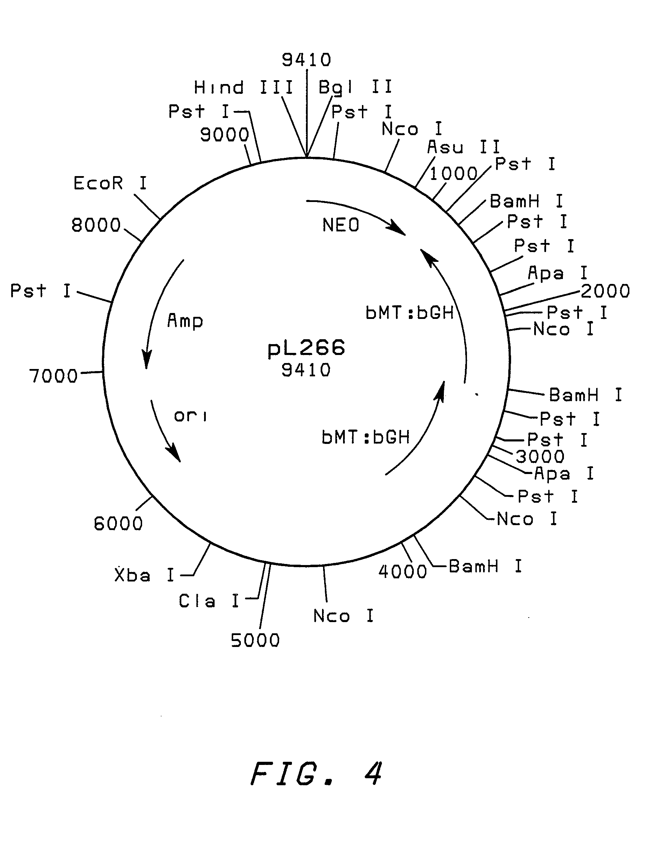

One suitable vector for the practice of this invention is the pL260 vector provided in Figure 3. It is preferred for high levels of production with this vector that 2-3 copies of a serial arrangement comprising a bovine metallothionein regulatory region operably linked to a heterologous DNA sequence (containing a 5' ATG start codon, a 3' stop codon and a nucleotide sequence which directs for polyadenylation) be present in the same orientation in this vector. These serial arrangements should be oriented such that the bMT-II regulatory region or mbMT-II regulatory region is operably linked to a heterologous DNA sequence and 3' thereto is linked at least one serial arrangement of a bMT-II regulatory region or mbMT-II regulatory region operably linked to a heterologous DNA sequence. A vector containing this type of serial arrangement in the same orientation is represented by pL266 provided in Figure 4.

Suitable host cells for the use of bovine metallothionein regulatory region include eukaryotic cells, particularly vertebrate cells, and preferably mammalian cells, including but not limited to bovine and murine cells.

Transformation of the bovine metallothionein regulatory region into a host cell line by a compatible vector may be accomplished by suitable transformation techniques known to those skilled in the art.

Expression of heterologous DNA sequences operably linked to a bovine metallothionein regulatory region transformed into a suitable host may be induced by introducing heavy metals into the host cell environment in a manner to facilitate host cell uptake to said heavy metals. Suitable heavy metals which will induce expression consist of heavy metals selected from the group consisting of Cd, Zn, Cu, Ag and Hg and combinations thereof. Preferred are combinations of Cd and Zn, optimally in a 1:4 ratio.

Additionally, it should be recognized that cells of different origins will respond differently to induction of expression with heavy metals. Human, porcine and monkey cells will adapt readily to heavy metals such as cadmium, whereas bovine cells may require significantly lower heavy metal concentrations initially to remain viable. Cells cultured over long periods of time in increasing concentration of heavy metals may also tolerate higher levels of heavy metals for induction of expression. These cell lines may also be removed from heavy metal concentrations prior to transformation and re-introduced into the presence of heavy metals after transformation for improved induction. Preferred, however, is the use of cell lines which have not been previously exposed to heavy metals. These cell lines demonstrate enhanced expression after transformation with vectors comprised of a bovine metallothionein regulatory region operably linked to a heterologous DNA sequence encoding polypeptide when they are exposed to heavy metals.

The following non-limiting examples are provided to illustrate the practice of the present invention.

## Examples

### General Information

### Strains and Cell Lines

MDBK (bovine kidney cell line) ATCC #CCL22
NIH 3T3 (mouse embryonic fibroblast cell line) ATCC #CRL1658
E. coli HB101 BRL #5B302
E. coli Y1088 NRRL #37195

### Media, Buffers, and Solutions

### Complete Media:

To a 500 ml bottle of Dulbecco modified Eagle media add the following:
1) 5 ml of penicillin -streptomycin solution from GIBCO final concentration = 10 $\mu$g/ml strep, 10 U/ml pen
2) 50 ml fetal bovine serum
3) 5 ml non-essential amino acids, final concentration = 1 mM
4) 5 ml glutamine, final concentration = 2mM

### 1M Tris buffer

121.1 g Tris base in 800 mL of $H_2O$; adjust pH to the desired value by adding concentrated (35%) aqueous HCl; allow solution to cool to room temperature before final pH adjustment, dilute to a final volume of 1 L.

### TE buffer

1.0 mM EDTA in 0.01 M (pH 7.4) Tris buffer

### SSC

0.15 M NaCl
15 mM sodium citrate adjusted to pH 7.0 with NaOH

### TAE

40 mM acetic acid
5 mM EDTA in 0.02 M (pH 8.3) Tris buffer

### Denhardt's solution (50x)

5 g Ficoll
5 g polyvinylpyrrolidone
5 g bovine serum albumin (BSA; Pentax Fraction V) brought to a total volume to 500 mL with water

### 20X SSPE

20 mM EDTA
0.16 M NaOH

0.2 M NaH₂PO₄•H₂O
3.6 M̄ NaCl adjusted to pH 7.0 with NaOH

dNTP stocks

0.5 mM dGTP
0.5 mM̄ dCTP
0.5mM̄ TTP
0.5 mM̄ dATP

Proteinase K buffer

0.01M Tris•Cl, pH 7.8
0.005M̄ EDTA
0.5% S̄DS

TBE

0.089M Tris•borate
0.089M̄ boric acid

Second Strand Buffer 2x

0.2M̄ HEPES, pH 6.9
20mM̄ MgCl₂
5mM dithiothreitol
0.14M̄ KCl
1mM̄ of each of the four dNTPs.

Nuclease S1 buffer, 10x

2M NaCl
0.5M̄ sodium acetate, pH 4.5
10mM̄ ZnSO₄
5% ḡlycerol

Tailing buffer, 2x

0.4M potassium cacodylate
50mM̄ Tris•Cl, pH 6.9
4mM̄ dithiothrietol
1mM̄ CoCl₂
2mM̄ [³H]dGTP (sp. act = 12 ci/mmole) 500 µg/ml bovine serum albumin

TBE gel

0.089M Tris
0.089M̄ boric acid
0.002M̄ EDTA

ETS buffer

10mM Tris pH 7.4

10mM EDTA
0.2% SDS

PBS

140mM NaCl
27mM KCl
10.3mM $Na_2HPO_4$
2.2mM $KH_2PO_4$

## Isolation and Characterization of the bMT-II Regulatory Region

## Example I

## Development of Cadmium Resistant Cell Line

A bovine kidney cell line (MDBK, ATCC#CCL22) was adapted to grow in 60 $\mu$M cadium by prolonged exposure of the cells to very low concentrations of cadmium, followed by growth in incrementally higher concentrations of cadmium. The adapted cell line responded to the increasing cadmium concentration by producing more BMT-specific mRNA, and was designated $MDBK^R$. The specifics of $MDBK^R$ development are as follows:

A vial of MDBK was introduced into a 75 $cm^2$ falcon flask in ~10 ml of complete media. The falcon flask was incubated at 37° C in 5-10% $CO_2$ atmosphere overnight. The media was changed the next day by aspirating off the old media and adding ~10 ml of new media to the flask. The cells were then allowed to grow at 37° C in 5-10% $CO_2$ until confluent, usually 3-4 days.

The cells were split when they become confluent. This was achieved by aspirating off the media and adding 2 ml of a solution of 1X trypsin•EDTA (GIBCO). This was aspirated off and replaced with 2 ml of the same solution. The cells were left in contact with this solution until they began to detach from the plate surface, usually 5-15 min. Once the cells were detached, 10 ml of complete media was added to the 2 ml solution of cells and the total volume was aspirated up and down to disperse the cell aggregates. All but 5-10% of the media-containing cells was aspirated out of the flask. 10 ml of media was added to the 5-10% cell solution remaining in the flask and the flask was again incubated at 37° C in 5-10% $CO_2$. This process was repeated every few days when the cells reached confluence.

To develop the cadmium resistant bovine kidney cell line, $MDBK^R$, the cells were seeded as described above on day 0 and 0.2 $\mu$M $CdCl_2$ was added to the medium. The cells were confluent on day 18, and they were split on day 28, and again on days 37, 44, and 47. The medium was changed every few days as needed, and supplementation with 0.2 $\mu$M $CdCl_2$ was continued with every medium change.

The cells were split again on day 52 and to one flask was added 2.0 $\mu$M $CdCl_2$. The other flask continued to receive 0.2 $\mu$M $CdCl_2$ supplementation. Both flasks were split on day 57 and all flasks were supplemented with 2.0 $\mu$M $CdCl_2$ at that time. On day 58 the cells were trypsinized and allowed to grow without $CdCl_2$. One day later, on day 59, the cells were again supplemented with 2.0 $\mu$M $CdCl_2$.

The cells were split on day 61 and the new flask was supplemented with 0.2 $\mu$M $CdCl_2$. One day later, day 62, the $CdCl_2$ concentration was increased to 2.0 $\mu$M. The cells were split three ways on day 63, and the $CdCl_2$ concentration in the three flasks were 2 $\mu$M , 2$\mu$M and 0.2 $\mu$M. The concentration of one flask was changed on day 64 from 2 $\mu$M to 5 $\mu$M. These three cultures were split on day 66 and the $CdCl_2$ concentrations remained as on day 64.

The 5 $\mu$M $CdCl_2$ cells were confluent on day 74. They were split and then split again on day 75. One flask of cells was grown in 10 $\mu$M $CdCl_2$ on day 75. The others remained at 5 $\mu$M $CdCl_2$. Ten days later, day 85, the 10 $\mu$M cells were split again and grown in 15 $\mu$M $CdCl_2$. These cells were split and the concentration of $CdCl_2$ increased as follows:

| day 91 | 20 μM |
| 121 | 30 μM |
| 147 | 40 μM |
| 177 | 50 μM |

On day 237 the cells were split and supplemented with 60μM CdCl₂ plus 80 μM ZnCl₂. The cells were maintained at 60 μM CdCl₂ + 80 μM ZnCl₂.

## Example II

## Construction of the MDBK$^R$ cDNA Library

### A. RNA Isolation

Poly A$^+$ RNA was isolated from MDBK cells adapted to growth in 50 μM CdCl₂ as follows. Approximately 10$^8$ cells growing in 50 μM CdCl₂ were trypsinized, collected by scraping into a 50 ml centrifuge tube and spun at 2,000 rpm for 10 min. The pellet was washed once with 1X PBS and then resuspended in 5 ml of 1X PBS. While lightly vortexing, 20 ml of Proteinase K buffer (containing 200 μg/ml of proteinase K) was added. The mix was incubated at room temperature for 30 minutes, and then extracted twice with phenol/chloroform:isoamyl alcohol and then once with chloroform:isoamyl alcohol (24:1). This step was followed by ethanol precipitation of the nucleic acids.

One-half volume phenol (recrystallized molecular biology grade phenol from Bethesda Research Laboratory, equilibrated with TE buffer) and one-half volume chloroform:isoamyl alcohol (24:1) was added to the RNA/DNA mix. The solution was shaken vigorously by hand for approximately 1 minute and then spun at 3,000 rpm for 15 minutes. The lower organic phase was removed and discarded. The extraction was repeated as above. An equal volume of chloroform:isoamyl alcohol (24:1) was then added and treated as above. The upper aqueous phase was collected and ethanol precipitated as follows. NaCl was added to a final concentration of 0.1 M along with two volumes of 95% ethanol. The solution was mixed vigorously and incubated at -20° C overnight. The precipitate was then collected by centrifugation at 3,000 rpm for 30 minutes.

The RNA and DNA precipitate was resuspended in 10 mls of TE buffer and layered onto two 4 ml cushions of CsCl (1 g/ml final concentration) in TE buffer in Beckman SW41 polyallomer tubes. The tubes were balanced with TE buffer and spun at 37,000 rpm for 20 hr at room temperature. The RNA pellet was collected by carefully aspirating off the supernatant. (The supernatant was removed down to the viscous DNA band.) The DNA was collected separately, ethanol precipitated, and stored for use later. Each pellet was resuspended in 3 ml of ETS buffer and then transferred to a centrifuge tube. The volume was increased to 15 ml with ETS buffer, and the tubes were gently heated at 65° C until the RNA went into solution, ~2 min. The RNA was then collected by centrifugation at 3,000 rpm for 30 minutes, and the pellet resuspended in 2 ml ETS buffer. The RNA concentration was then determined by measuring absorbance at 260 nm, and adjusted to a concentration of <5 mg RNA/ml with ETS. This solution was then incubated at 65° C for 5 minutes, cooled with ice to room temperature, and 5M NaCl was added to a final concentration of 0.5M. The RNA was applied to an oligo dT column prepared with 2g oligo dT cellulose (Collaborative Research) in a 1 ml column. Prior to the addition of the RNA, the column was packed in ETS buffer, washed with 0.5M NaOH, and equilibrated with 0.5M NETS (ETS buffer plus 0.5M final concentration NaCl). The flow through was caught and reloaded onto the column twice, which was then washed with 30 ml of 0.5M NETS. The polyA$^+$ RNA was eluted with 4 ml of ETS buffer, ethanol precipitated with two volumes of ethanol plus NaCl to a final concentration of 0.25M, and kept at -20° C overnight. The RNA precipitate was collected by centrifugation at 10,000 rpm for 30 minutes using siliconized 15 ml Corex tubes.

The pellet was resuspended in 100 μl of ETS buffer, and 5 μl of this was used to determine the concentration of polyA$^+$ RNA. 1 μg of the polyA$^+$ RNA was electrophoresed on a 1.5% agarose gel using 1X TBE (tris-borate-EDTA) running buffer to determine if the RNA was intact. The remaining polyA$^+$ RNA was precipitated by the addition of two volumes of ethanol plus 0.25M NaCl, and kept at -20°C overnight. The RNA precipitate was collected by centrifugation at 3,000 rpm for 30 minutes. The RNA pellet was resuspended to a final concentration of 1 μg/μl with sterile distilled water.

## B. Production of cDNA

The cDNA library was constructed as follows. 5 μl of the RNA solution (5 μg RNA) was combined with 1.2 μl of 10 mM methyl mercury hydroxide (Alfa Biochemicals) and the mix was kept at room temperature for 5 minutes. 2.0 μl of 0.7 M β-mercaptoethanol, 0.5 μl of 100 μM DTT and 3 μl of RNase (24 U/ml; Promega Biotec) were added, and the reaction was kept at room temperature for 5 minutes. The following reagents were added to the above mix and the final reaction (which was now 50 μl) was kept at 42°C for 60 minutes.

| | |
|---|---|
| 23.3 μl | reverse transcriptase buffer |
| 5.0 μl | actinomycin D (600 μg/ml) |
| 2.5 μl | $^{32}$P-dCTP (3200 Ci/mmole) |
| 8.3 μl | reverse transcriptase (20 U/ul; (Molecular Genetics Resources) |

Prior to the addition of reverse transcriptase, a 0.5 μl sample was taken as a time 0 sample for TCA precipitation.

Two 0.5 μl samples was taken for time 60, then the reaction was stopped by the addition of 0.5 μl 20% SDS, 2 μl 0.5M EDTA, and 21.7 μl 1N NaOH and held at 65°C for 30 minutes. Then, 10 μl of 1M Tris/pH 7.4 and 21.7 μl 1N HCl were added and the mix was phenol/chloroform:isoamyl alcohol extracted once the chloroform:isoamyl alcohol extracted once as described above.

TCA precipitations were performed on the two 0.5 μl reaction samples from time 0 and time 60 minutes. The TCA procedure was as follows. Each 0.5 μl sample was placed in 100 μl final volume [80 μl TE buffer, 20 μl carrier RNA (10 mg/ml)]. 10 μl was removed and spotted onto a nitrocellulose disc. The remaining 90 μl were precipitated by adding 400 μl of 20% TCA, and then placed on ice for 10 minutes. The precipitate was collected by filtering onto a nitrocellulose disc and then washing with 5% TCA at room temperature. All filters were dried under a hot lamp and each was placed in scintillation fluid for counting (cpm). The percent incorporation of label into high molecular weight DNA was determined by using the 10 μl and 90 μl values. 1 μg of ss cDNA equaled approximately 8.5% incorporation.

A 1.5% agarose TBE gel was run with 0.5 μl of reaction product. The average size of ss cDNA was shown to be ~1500-2000 nucleotides. The top aqueous phase removed from the extractions was applied to a 1 ml Sephadex G50 column in TE buffer to remove unincorporated label. TE buffer was used for elution and 100 μl fractions were taken. Fractions containing labeled ss cDNA were pooled. The fraction pool was butanol extracted to reduce volume to approximately 50 μl, and 10 μl of 10M NH₄OAc and 2 volumes of ethanol were added. The mix was kept at -20°C overnight. (For the butanol extraction, six volumes of butanol were added to one volume of solution and shaken vigorously. The top aqueous phase was removed and discarded).

The ss cDNA precipitate was collected by centrifugation in a microfuge for 15 minutes. The DNA pellet was washed once with 70% ethanol. It was dried down completely in a speed vac and the pellet was counted (to provide a baseline from which to determine subsequent losses of radioactive label). The second strand was synthesized by combining the following and keeping the reaction at 14°C for 2 hours and then 37°C for 20 minutes:

| | |
|---|---|
| | ss DNA pellet |
| 25 μl | 2X second strand buffer |
| 22 μl | dH₂O |
| 1.0 μl | $^{32}$P-dCTP (3200 Ci/mmol) |

Two 0.5 μl samples of reaction mix were taken as time 0 for a TBE gel and TCA precipitation as above. Then 2.0 μl Klenow (5U/μl; IBI) was added and the reaction allowed to proceed at 37°C for 20 minutes.

Two 0.5 μl time = 160 minutes samples were taken for TCA precipitation and TBE gel, and then the reaction was stopped by the addition of 2 μl 0.5M EDTA. The reaction was subject to one phenol/chloroform:isoamyl alcohol extraction and one chloroform:isoamyl alcohol extraction. A TCA precipitation was performed on the 0.5 μl samples as described previously. A 1.0% formaldehyde gel was then run with this sample. An overnight exposure showed the ds cDNA to be of the correct size, i.e. larger than ss cDNA. The sample was then run over a Sephadex G-50 column and 200 μl fractions were collected at first, followed by 100 μl fractions. TE was used to elute. Fractions were pooled and butanol extracted to 50 μl. 10 μl of 10N NH₄OAc was added and the sample was then ethanol precipitated overnight with 2 volumes of ethanol.

The precipitated double-stranded cDNA was spun down in a microfuge for 15 minutes and the pellet was resuspended in 50 μl of TE buffer, 10 μl NH₄OAc, and two volumes of ethanol. The mix was incubated in a dry ice/ethanol bath for 10 minutes and then centrifuged for 15 minutes in a microfuge. The pellet was washed with 70% ethanol and spun again for 15 minutes. The ethanol was drawn off and the pellet was dried briefly in a speed vac.

A reverse transcriptase reaction was performed to complete the second strand. The pellet was resuspended in 26 μl dH₂O and the following were added and allowed to react at 42° C for 60 minutes:

5.0 μl 0.5 M Tris/pH 8.3
2.5 μl 0.2 M MgCl₂
2.0 μl 0.7 M β-mercaptoethanol
6.5 μl 1 M KCl
1.25 μl of 20 mM dNTPs (all 4; 1.25 μl of each)
3.0 μl of reverse transcriptase (18 U/μl)

The reaction was stopped by the addition of 2 μl of 0.5M EDTA, followed by one phenol/chloroform:isoamyl alcohol extraction and one chloroform:isoamyl alcohol extraction.

10 μl of 10M NH₄OAc was added and the mix was ethanol precipitated at -20° C overnight. The next day the DNA precipitate was collected by centrifugation in a microfuge for 15 minutes. Following a 70% ethanol wash, the DNA was dried in the speed vac. An S1 nuclease reaction was then performed by resuspending the pellet in 44 μl dH₂O, combining the following reagents, and incubating at 37° C for 30 minutes:

| 5 μl | 10X S1 buffer |
| 1 μl | S1 (stored at 4° C; Sigma; 10 U/μl |

Prior to the addition of S1, two 0.5 μl samples were taken for time 0 TCA precipitation and a 1.5% aqueous gel. The reaction time was stopped by the addition of 2 μl of 0.5M EDTA. The reaction was phenol/chloroform:isoamyl alcohol extracted once and chloroform:isoamyl alcohol extracted once. Prior to stopping the reaction two 0.5 μl time 30 samples were taken for TCA precipitation and a 1.5% aqueous gel.

The aqueous phase was supplied to a G-50 column and 100 μl fractions were collected by TE buffer elution. Approximately five cDNA-containing fractions were pooled and butanol extracted down to a 50 μl final volume. 10 μl of NH₄OAc plus 2 volumes of ethanol were added and the double stranded cDNA precipitate was collected by incubation at -20° C overnight. The precipitation DNA was spun down and washed with 70% ethanol, followed by drying in the speed vac. The pellet was resuspended in 40 μl dH₂O.

One half of the cDNAs were C-tailed by combining the following and holding at 37° C for 30 minutes:

| 20 μl | cDNA (100 ng/20 μl) |
| 10 μl | 5X tailing buffer |
| 3 μl | 10 μM cold dCTP |
| 1 μl | ³²P-dCTP |
| 15 μl | dH₂O |
| 1 μl | terminal transferase, TTase (21 U/μl; Ratliffe Biochemicals) |

0.5 μl of the reaction were taken at time 0 and 30 minutes and a TCA reaction was performed. The TCA reaction showed that only a few C's were added, so the reaction was taken off ice and 3 μl 10 μM CTP and 1 μl TTase were added. The reaction proceeded for 30 minutes at 37° C. A second TCA precipitation showed the appropriate number of C's were added.

The tailed cDNA was run over a Sephadex G-50 column, fractions were collected by TE buffer elution, and then pooled. After butanol extraction, the cDNAs were ethanol precipitated at -20°C overnight. The precipitated C-tailed cDNA was collected by centrifuging in a microfuge for 15 minutes and then washing with 70% ethanol. The cDNA was dried and resuspended in 20 μl TE.

The tailed cDNAs were annealed to a G-tailed pBR322 vector as follows:

| 19 μl | cDNA (100-150 ng) |
|---|---|
| 5 μl | 5X annealing buffer |
| 1.0 μl | vector (New England Nuclear pBR322; diluted 1:3) |

The above components were combined and held at 65°C for 3 hours, and then 42°C for 2 hours. The reaction was allowed to cool to room temperature and then stored at -20°C. 100 μl of competent HB101 (BRL #5B302) cells were transformed with 16 ng/2 μl of the cDNA vectors above following manufacturer's instructions. Approximately 1,000 colonies were generated.

## Example III

## Screening and Characterization of the MDBK<sup>R</sup> cDNA Library

The bovine cDNA library was screened in duplicate with a mixture of three oligonucleotides, (85-47, 85-48, 85-53; see Table 3 below), which code for the 5′ end of the monkey MT-I sequence, the 5′ end of the monkey MT-II sequence, and the antisense sequence of the middle portion of the consensus metallothionein sequence, respectively; and with a cloned fragment coding for monkey metallothionein (L142-1).

## Table 3

## Oligonucleotide Probes

**85-47[1]**

5'-AA-ATG-GAC-CCC-AAC-TGC-TCC-TGC-GCC-ACT-GGT-CTC-T-3'

**85-48**

5'-ATC-GAT-CCC-AAC-TCG-TCT-TGC-GTC-GCC-GGT-GAC-T-3'

**85-53**

5'-GCT-TTT-CTT-GCA-GGA-GGT-GCA-TTT-GCA-CTC-TTT-GCA-3'

[1] Complete sequence published by Searle et al. Mol. Cell. Biol. 4:1221-1230 (1984).

Duplicate lifts of the library were prepared and each filter was prehybridized for 1.5 hour with 5X SSC, 0.1% SDS, and 10X Denhardt's at 42°C. The three oligos were kinased and the L142-1 insert was labeled by random priming (5 x 10⁵ cpm/μl). The prehybridized filters were washed briefly in 5X SSC and to one was added the mixed oligo probe (25 μl of each oligo) and to the other was added the L142-1 probe (10 μl). 5X SSC, 0.1% SDS, 10X Denhardt's and sonicated salmon sperm DNA to a final concentration of 500 μg/ml was added and hybridization continued overnight at 54°C.

The filters were washed at 65°C four times for 15 minutes each time in 150 ml of a 0.1X SSC plus 0.1% SDS solution. The filters were then exposed to X-ray film and an autoradiograph of each was developed. Six positives, A-F, were identified that hybridized to both of the probes and were further characterized as described below.

Minipreps were performed on positives A-F. PstI restriction digests were done following manufacturer's instructions and analyzed on a 1% agarose gel. Of the six positives, clone MDBK-C′ was chosen. The mini-prepped DNA was probed with the mixed oligos as above. Prehybridization was at 54°C for 3 hours (5X SSC, 0.1% SDS, 10X Denhardt's). Hybridization with probe was continued overnight using the same conditions. Clone MDBK-C′ again hybridized to the probe. It was renamed L219-1.

The insert from clone L219-1 was restriction mapped. An approximate 450 base pair PstI/BamHI fragment of L219-1 hybridized to the 5′- and 3′-specific oligo probes. The insert from L219-1 was subcloned into pUC19, yielding clone L220-1.

## Example IV

### Sequence of L220-1 Insert

The insert of L220-1 was sequenced by the dideoxy method [Sanger et al., PNAS 74, 5463 (1977)]. The DNA sequence was translated into its deduced amino acid sequence and it was found to encode a protein which was identical to the published amino acid sequence of bMT-II except for one amino acid substitution. The codon for aa #18 in L220-1 encodes a proline, while the published sequence for bMT-II has a serine in aa #18 position (Mungen et al. JBC 260:10032-10038 (1985)

## Example V

### Construction of the MDBK Genomic Library

DNA was extracted from MDBK and MDBK^R cells as described in Example II. The DNA was resuspended in TE buffer and 15 μg of DNA was digested with PvuII, electrophoresed on a 1.0% agarose gel using TBE buffer, transferred to a zeta probe nylon membrane, and probed with a kinased 36 mer oligonucleotide probe spanning the ATG of the bMT insert in L220-1. The sequence of the oligo is as follows:

5′-CGC-GGT-GCA-GGA-GCA-GTT-GGG-ATC-CAT-GGC-GAG-CTG-3′

Hybridization conditions were as follows:

| Hybridization:<br>(17hr./42°C) | 6X SSPE<br>5X Denhardt's<br>25% Formamide | Final<br>Wash: | 1XSSPE<br>0.1%SDS<br>62°C |
|---|---|---|---|
| | 0.1% SDS<br>200 μg/ml sonicated herring sperm DNA | | |

The filter was dried and placed under film for 86 hours. The developed film clearly showed a PvuII fragment of 4.7kb in size that was amplified in the MDBK[R] cells in comparison with the MDBK cells, which presumably contained the bMT gene locus. A large scale fragment prep of genomic DNA was performed to isolate the 4.7 kb PvuII fragment. It was digested with PvuII and size-selected on a 0.8% agarose gel. Lambda size markers were run in parallel. The gel was laid on a light box and that section of the gel containing fragments of 3.5-6 kb in size was excised with a razor blade and placed in dialysis tubing. The DNA fragments were electroeluted out of the gel slice contained in the dialysis tubing, and the DNA was collected from the solution in the tubing. It was purified by running it over a Schleicher and Schull elutip column, following manufacturer's instructions.

In preparation for insertion into λgt10 and λgt11 cloning vectors, EcoRI linkers were added to the ends of the size-selected fragments.

The following linkers were used:

18 mer 5'CCTTGACCGTAAGACATG-3'
22 mer 3'GGAACTGGCATTCTGTACTTAA-5'

The 18 mer was phosphorylated by combining the following reagents and incubating at 37°C for 15 min.:

| 225 pmoles 18 mer | + |
|---|---|
| water | 6.8 μl |
| 10X kinase buffer | 1.2 μl |
| 32P-γATP (7000 Ci/mmole) | 1.0 μl |
| kinase 2 (U/μl) | 1.0 μl |
| | 10 μl |

The following two reagents were added to the above mixture and incubated at 37°C for 30 min.:

| 10 mM ATP | 1 μl |
|---|---|
| kinase (2 U/μl) | 1 μl |

The enzyme was then inactivated by boiling for 10 min. The 22 mer was hybridized to the phosphorylated 18 mer by addition of 225 pmoles of the 22 mer (plus water to bring volume to 15 μl), and incubated at 65°C for 5 min. The reaction was then allowed to slow cool to room temperature.

The adapters were present at a concentration of 15 mmoles/μl, and were ready for genomic cDNA-vector ligation.

The following were combined:

| genomic DNA | + |
|---|---|
| hybridized adapters (15 pmol/μl) | 50-fold molar excess over genomic DNA |
| water | 16 μl |
| 10X ligase buffer | 2 μl |
| ligase (10 U/μl) | 2 μl |
| | 20 μl |

The linkered genomic DNA was phosphorylated using the following technique. The ligase was inactivated by heating the mixture to 72°C for 15 min. The following reagents were then added to the ligation reaction and heated at 37°C for 30 min.:

| | |
|---|---|
| ligation reaction | 20 µl |
| water | 24 µl |
| 10X kinase buffer | 3 µl |
| 10 mM ATP | 1 µl |
| kinase (2 U/µl) | 2 µl |
| | 50 µl |

The reaction was stopped by the addition of 2 µl 0.5M EDTA, followed by one phenol/chloroform extraction and one chloroform extraction.

The above-described DNA was purified and size-selected using the following techniques. The DNA was dried down in a speed vac to ~20 µl. 2.5 µl of gel loading dye was added, and the sample ran over a BIO GEL A-50 column that had been washed with TE buffer. Five minute fractions were collected (~30 µl) and counted in a scintillation counter. 0.5 µl of several of the collected fractions were run on a 1.5% agarose minigel. The gel was photographed, dried down, and placed under film.

The EcoRI-ended fragments were ligated into λgt11 using the following procedures. The fractions containing DNA were pooled, butanol extracted down to 20-30 µl, and 5 µl of 10M NH₄OAc plus two volumes of ethanol was added to precipitate the DNA. The sample was spun in a microfuge for 15 min., and then subjected to a 95% ethanol wash and dry. The DNA was resuspended in TE buffer (~0.10 pmol/µl).

The ligation reaction contained the following, which was incubated at 14-16° overnight:

(use 1 µg of λgt11 vector = 0.035 pmol vector)

| | | |
|---|---|---|
| λgt11 (1 µg/µl) | 1 µl | |
| DNA insert | | (2-4 fold molar excess of DNA over vector) |
| water | to 3 µl | |
| 5X ligase buffer | 1 µl | |
| ligase (10 U/µl) | 1 µl | |
| | 5 µl | |

λgt10 and λgt11 were packaged using the Gigapack in vitro packaging kit supplied by Stratagene, following manufacturer's instructions. The λgt11 phage were then placed with the E. coli host Y1088 on five plates at a concentration of ~20,000 plaques per plate.

## Example VI

## Screening Genomic Library

Two probes were made for screening the MDBK$^R$ genomic library for the bMT-2 regulatory sequence. The 36 mer oligo probe for Example V was kinased to a specific activity of 5 x 10$^8$ cpm/µg. A large scale plasmid prep was made of pL220-1 from Example III. The plasmid was nick-translated to a specific activity of 3 X 10$^8$ cpm/ml. Duplicate lifts were made of each plate.

One set of lifts was hybridized to the oligo in a mixture of 6x SSPE, 5x Denhardt's, 25% Formamide, 0.1% SDS, and 200 µg/ml sheared herring sperm DNA, and ~1 x 10$^6$ cpm/ml of probe for 17 hours at 42°C. The filters were then washed several times, for 15 minutes each wash, in a mixture of 0.2x SSPE and 0.1% SDS, at 55°C. The filters were placed under film with a screen overnight.

The duplicate set of filters was hybridized with the plasmid probe following the conditions specified for the oligo, except 5x SSPE and 50% Formamide were used instead of 6x SSPE and 25% Formamide. The

16

filters were washed several times in 0.1x SSPE and 0.1% SDS at 65° C and placed under film with a screen overnight.

The films of the duplicate lifts were developed and the spots of hybridizations, representing λ clones comprised of insert sequences homologous to the oligo and plasmid probes, were compared. There were 12 potentially positive clones identified that hybridized to the ATG-spanning oligo. Hybridization with the plasmid pL220-1 produced a heavy generalized background making it impossible to identify clones containing sequences of the bMT promoter element with this probe. The background was due to homology between sequences in the probe and sequences in the λgt11 vector plus sequences in the E. coli Y1088 host.

Two clones, λBMT9 and λBMT12, were chosen for additional characterization. These clones were plaque purified and minipreps were performed as described in Benson et al., Biotechniques, May/June 1984, p. 126. The insert sizes of clones λBMT9 and λBMT12 were determined by digesting the miniprep with EcoRI to excise the insert, and running the disgestion products on a 0.8% agarose gel. ØX174 HaeIII-digested DNA and λ HindIII-digested DNA size markers were run in parellel. Ethidium bromide staining showed both inserts to be 4.7 Kb in size. Another aliquot of first-digested miniprep DNA was run on a second 0.8% agarose gel, and both agarose gels were completely dried. After denaturation and neutralization of the nucleic acids the gels were probed with either the ATG-spanning oligo or pL220-1 as previously described. Both inserts hybridized to both probes.

A second miniprep was performed for λBMT9. A restriction map was generated by digesting aliquots of the miniprep DNA with the following enzymes: BamHI, BglII, HindIII, KpnI, NcoI, PstI, SacI, XbaI, following manufacturer's instructions. The restriction map of λBMT9 is shown in Figure 1.

## Example VII

## Sequencing λBMT9 Insert

A large scale prep of λBMT9 was performed and insert DNA was excised by EcoRI digestion. The insert was purified by electrophoresis in a 0.8% TBE agarose gel. The 4.7 kb fragment was cut out of the gel and the fragment was electroeluted in dialysis tubing. The fragment was then purified by an elutip column. Various subclones of the insert were constructed in M13, and the ~1740bp HindIII - EcoRI fragment was then sequenced following the Sanger dideoxy sequencing method (Sanger, F., and A. R. Coulsoun, 1975, "A Rapid Method for Determining Sequences in DNA by Primed Synthesis with DNA Polymerase" J. Mol. Biol. 94:441-448).

The sequence of the λBMT9 insert (Table 1) was compared to known sequences of metallothionein promoter elements from other species. The identification of multiple metal regulatory regions, "TATA" box, and an initiator ATG identified the λBMT9 insert as containing a MT promoter element. The comparison of the sequence for the structural gene of λBMT9 with the published amino acid sequence of the bMT-II protein, identified the λBMT9 insert as coding for the bMT-II promoter element and a portion of the structural gene.

## Example VIII

## Construction of pL260

Plasmid pL17-1 is a pBR322-based plasmid which contains both the 5′ and 3′ MSV LTRS. The 5′ LTR-containing fragment was excised from pL17-1 by first preparing a large scale plasmid prep (Maniatis, et al.) and then digesting with EcoRI and PstI following manufacturer's instructions. The correct sized fragment was identified on and isolated from a 1% agarose gel. The approximately 1680 bp LTR-containing fragment was ligated into pUC8 plasmid DNA, which has previously been cut with PstI and EcoRI. Ligation conditions were as follows. ~100 ng of each fragment was put in a ligation reaction mixture with T4 ligase at 14° C overnight. The ligation products were transformed into bacterial HB101 cells. The transformants were selected for by Amp$^R$. DNA minipreps were prepared on transformants, and restriction digests with diagnostic enzymes confirmed the correct construction of the plasmid. The resultant plasmid was called pL153.

Plasmid pL38 is a recombinant retroviral vector designed to express chicken growth hormone (cGH). The 5' LTR is from MSV and is used to promote expression of the cGH gene. The bacterial neomycin gene, neo, is employed by the plasmid as a selection marker in eukaryotic cells. Its expression is regulated by the mouse metallothionein promoter (mMT-I). The 3' LTR element is from MLV and its presence in the vector, along with the 5' LTR, allows random, single copy integration of the plasmid into the eukaryotic genome. A large scale plasmid prep of pL38 was performed. The DNA was digested with HindIII and BamHI and the approximately 3220 bp smaller fragment was isolated. Contained on this fragment is the mMT-neo expression unit. A second digest using BamHI and EcoRI yielded a fragment of ~4260 bp which contained the bacterial origin of replication (ori), the bacterial ampicillin resistance gene (amp), and the MLV LTR.

A large scale plasmid prep of pL153 was performed and the MSV LTR-containing fragment was excised by EcoRI and HindIII digestion. The correct sized fragment, approximately 1200 bp, was identified on, and isolated from an agarose gel.

The three isolated fragments were ligated together using standard ligation conditions. Bacterial cells were transformed with the ligation mixture and then grown in ampicillin-containing media. Transformants were identified by their ability to grown in ampicillin. Plasmids were recovered from transformants and diagnostic digests confirmed correct plasmid assembly. A HindIII and BamHI digest yielded two bands of 5499 and 3215 bp; a HindIII and BamHI/EcoRI digest yielded bands of 4310, 3184, 1189, 31 (not visible). The resultant plasmid was called pL156.

Plasmid pL156 contained the following in clockwise orientation: the MSV LTR, mMT-neo, MLV LTR, bacterial ori, and bacterial amp. Two separate digestions were performed on a large scale plasmid prep of pL156 to isolate the mMT-neo expression unit and the plasmid sequences plus the LTRs, respectively. First, a HindIII and AsuII double digest was performed. Two fragments resulted from this digestion. One was approximately 5660 bp and contained the 5' MSV LTR and the 3' MLV LTR. This was isolated from an agarose gel. An EcoRI/AsuII double digest of pL156 was also performed and resulted in three fragments. The intermediate-sized fragment, approximately 3,000 bp, contained the mMT-neo expression unit. It was identified on, and isolated from, an agarose gel.

Plasmid pL223 is a pUC9-based plasmid containing bovine growth hormone (bGH) cDNA sequence. A large scale plasmid prep of pL223 was performed and the bGH cDNA was excised from pL223 by an EcoRI/HindIII double digest. Two fragments result from this digestion. The smaller, approximately 900 bp fragment contained the bGH sequence. It was identified on, and isolated from an agarose gel.

A three-way ligation was performed with the EcoRI/HindIII fragment, the EcoRI/AsuII fragment, and the AsuII/HindIII fragment using standard ligation conditions. Bacteria were transformed with the resulting plasmid, pL239, and ampicillin resistant transformants were selected. pL239 was the only possible ligation product, and it was identified by diagnostic digestion of miniprepped DNA. Plasmid pL239 contains the following in clockwise orientation: the 5' MSV LTR, the bGH cDNA, the mMT-neo expression unit, the 3' MLV LTR, and the bacterial ori and amp gene.

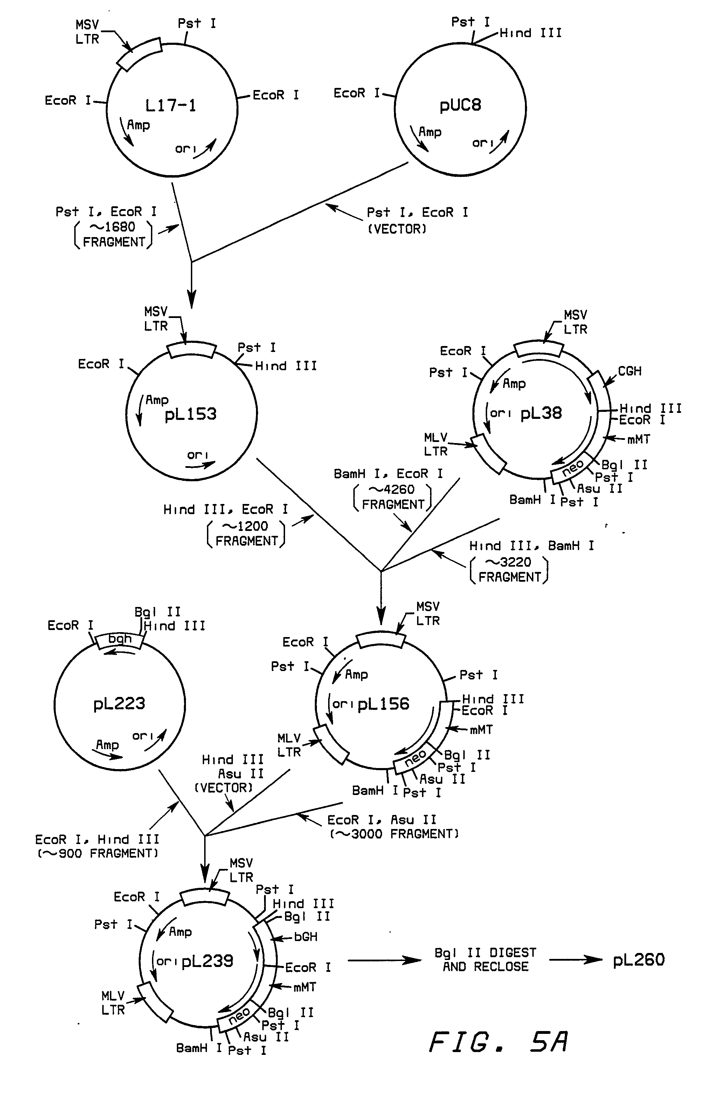

The vector pL260 was constructed from pL239 by excising out the bGH gene and the mMT promoter as follows. A large scale plasmid prep of pL239 was prepared and digested with BglII which yielded two fragments. The larger vector fragment, approximately 6750 bp, was identified on, and isolated from, an agarose gel. The linear vector was circularized with DNA T4 ligase. The resultant plasmid, pL260, was confirmed from miniprepped DNA from amp$^R$ bacterial cells that had been transformed with the plasmid. It is comprised of the 5' MSV LTR, the bacterial neo gene, and the 3' MLV LTR, along with the bacterial amp gene and ori. Plasmid pL260 (Figure 3) is approximately 6700 bp. A representational flow chart of this contruction is provided in Figure 5A.

## Example IX

### Construction of pL264, pL265, and pL266

The following manipulations were performed to insert the bMT-bGH sequence into the generalized recombinant expression vector pL260 from Example VIII.

Plasmid pBGH10A is a pBR322-based plasmid comprised of the bGH cDNA. A partial PstI digestion of a large scale plasmid prep of pBGH10A yielded several different fragments, one of which was approximately 910 bp and contained the bGH sequence minus nine bp of 5′ coding sequence. This fragment was identified on, and isolated from, an agarose gel. Plasmid pUC9 was linearized by PstI digestion and then isolated from a gel. A standard ligation of PstI-digestion pUC9 and the PstI-ended bGH fragment was performed. Bacteria were transformed with the ligation mix and amp^R colonies were selected. Diagnostic digests of miniprepped DNA identified the correct construct, named pL187. An Apal/PstI digest yielded the vector fragment plus a 332 bp band. A HindIII/EcoRI digest yielded the vector plus a 900 bp band.

Plasmid pL187 is comprised of pUC9 plasmid sequences, including the bacterial amp gene and ori, and the almost complete coding sequence of bGH. The orientation of the bGH fragment in pL187 is opposite from that in pBGH10A.

The missing 5′-bGH sequence was replaced by the following three way ligation. The 5′-end of the bGH gene in pL187 was isolated as an approximately 315-bp PstI/Apal fragment by digestion of a large scale plasmid prep of pL187 with those enzymes. The correctly sized fragment was identified from the several PstI/Apal fragments evident on a gel and isolated. A HindIII/Apal digest was performed on prepped pL187 and the large, approximately 3180 bp fragment was identified and isolated. This fragment contains most of the pL187 plasmid sequence. The PstI/Apal and Apal/HindIII fragments were used in a three-way ligation with a double stranded synthetic oligonucleotide. The oligo served to add back the missing 5′-bGH sequence and generate two more cloning sites at the 5′ end of the bGH gene (BglII and Ncol), in addition to the already existing HindIII and PstI sites. The ligation conditions were standard. The sequence of the synthetic oligonucleotide pair was:

<u>HindIII</u>          <u>PstI</u>

5′  AGCTTAGATCTCC|ATG|GCTGCA 3′

ATCTAGAGG TAC GC

Bacteria (MC1061) were transformed with the ligation mix and amp^R colonies were selected. The resultant plasmid was called pL223 and was identified by diagnostic digest of miniprepped DNA. (Digestion with HindIII, Ncol, or BglII linearized the correct vector). Plasmid pL223 contained the complete coding sequence for bGH.

The bMT regulatory region is contained in λgt10-based plasmid λBMT9. The bMT fragment was removed from λBMT9 by an Ncol digest of a large scale plasmid prep of λBMT9. The approximately 680 bp fragment was identified on, and isolated from, an agarose gel.

A large scale plasmid prep of pL223 was digested with Ncol, which opened the plasmid at the 5′-end of the bGH gene. The linearized plasmid was isolated from an agarose gel. A ligation was performed with the two Ncol-ended fragments to inset the bMT promoter 5′ of the bGH gene. Bacterial cells MC1061 were transformed with the ligation product and amp^R colonies were selected. Plasmid DNA was isolated from transformed cells by miniprep and the orientation of the bMT insert was verified by restriction digest of miniprepped samples of several clones. One plasmid which assembled in the correct orientation was pL254.

Plasmid pL254 is comprised of a bMT-bGH expression cassette. BamHI digestion releases a fragment containing the 5′-end shortened bMT promoter functionally oriented to express the complete bGH gene sequence. Such a digestion was performed on a large scale plasmid prep and the correctly sized fragment, approximately, 1250 bp, was identified and isolated from a gel. The BamHI-ended fragment was then ligated into plasmid pL260 that had been opened at the 3′-end of the neo gene by digestion of a large scale plasmid prep with BamHI. The ligation product was transformed into bacterial MC1061, and amp^R colonies were selected.

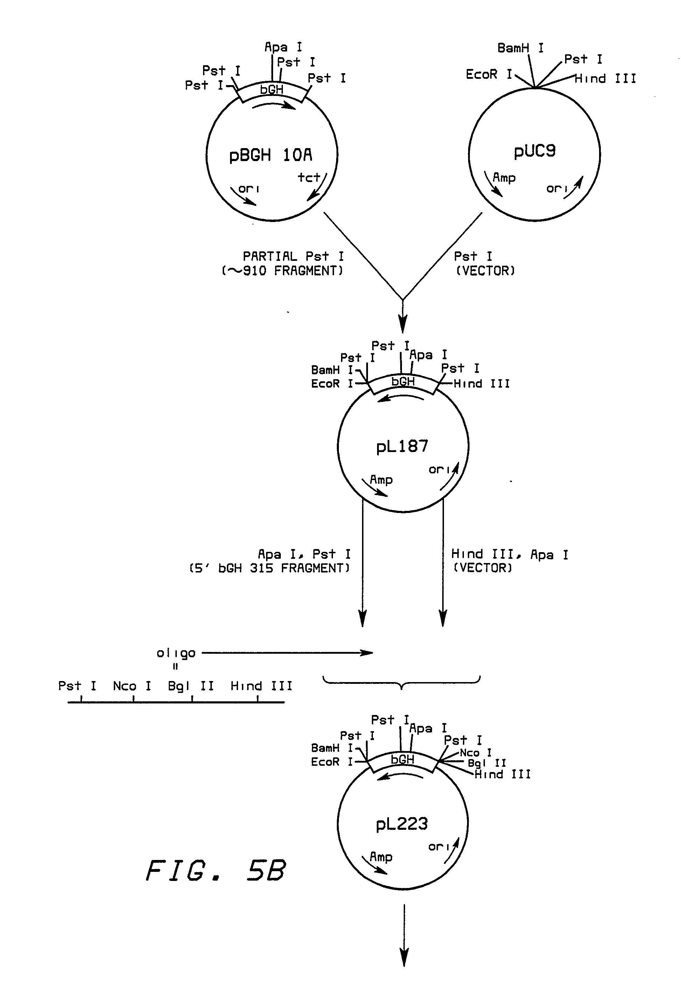

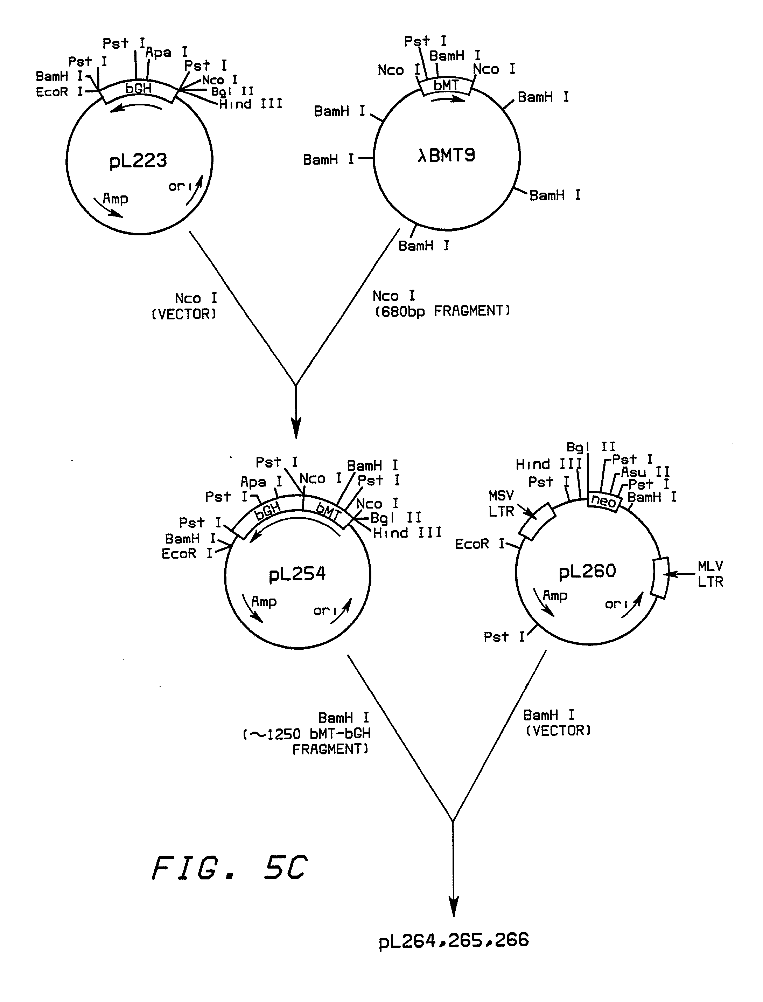

Three types of plasmids were isolated: pL264, pL265, and pL266 (Figure 4). Plasmid pL264 carries the bMT-bGH expression unit in the same orientation as the neo gene; the expression unit is in the opposite orientation in pL265. Plasmid pL266 carries two copies of the bMT-bGH expression unit, in tandem, in the opposite orientation as the neo gene. A representational flow chart of these constructions is provided in Figure 5B and 5C.

**Example X**

19

## bMT-II Promoter Activation Conditions

### A. Cell Culture

Three cell lines were employed:

1) MDBK cells - a bovine kidney cell line, ATCC #CCL22,

2) MDBK$^R$ cells, as described in Example I,

3) NIH3T3 cells - a mouse embryonic fibroblast cell line, ATCC #CRL1658.

All three cell lines were grown in the following manner. A vial of cells was introduced into a 75 cm$^2$ falcon flask containing ~10 ml of complete media. The flask was incubated at 37°C overnight in 5-10% $CO_2$. The media was changed the next day, and the cells allowed to grow at 37°C in 5-10% $CO_2$ until confluent, approximately 3-4 days.

The cells were then split when they become confluent. This was achieved by aspirating off the media and adding 2 ml of a solution of 1X trypsin•EDTA (GIBCO). This was aspirated off and replaced with 2 ml of the same solution. The cells were left in contact with this solution until they begin to detech from the plate surface, usually 5-15 minutes. Once the cells were detached, 10 ml of complete media was added to the 2 ml solution of cells and the total volume was aspirated up and down to disperse the cell aggregates. All but 5-10% of the media containing cells was aspirated out of the flask. 10 ml of media was added and the flask was incubated at 37°C in 5-10% $CO_2$. This was repeated every few days as the cells reached confluency.

### B. Transfection of Cells in Culture

Tissue culture cells wre split to 60 mm dishes at a concentration of ~5 x 10$^5$ cells/dish 24 hours prior to transfection. 3 ml of complete media was added, and the cells were incubated overnight at 37°C. 2 to 3 hours before transfection the media was changed by aspirating off the old media and adding 3ml of fresh media.

A calcium phosphate precipitate was then prepared with each plasmid as follows. 10 µg of purified plasmid DNA and 400 µl total volume of precipitated DNA solution was added per 60 mm dish.

| Transfection Mixture | | | |
|---|---|---|---|
| Tube 1 | | Tube 2 | |
| Salmon sperm | | | |
| DNA (1 mg/ml) plasmid or DNA 2.5 M CaCl₂ distilled H₂O | 10 µl 10 µg 20 µl 170 µl | 2X HBS (GIBCO) | 200 µl |
| Total | 200 µl | Total | 200 µl |

Each tube was prepared (usually 15 ml Falcon tubes) separately, in a laminar flow hood. While slowly bubbling a stream of air into the tube containing the DNA-CaCl₂, the HBS solution was added in a dropwise manner. The resultant Ca₃(PO₄)₂ solution was then allowed to incubate at room temperature for 15-30 minutes. The 400 µl of precipitated DNA solution was then added to a 60 mm dish, still containing 3 ml of media. After swirling slightly to evenly distribute the solution, the dish was incubated at 37°C for 6 hours. After 6 hours, the media was changed and replaced with fresh complete media. After 48 hours, cells were split to a 100 mm dish and G418, a neomycin analog, was added at the following concentrations:

3T3 cells - 400 µg/ml

MDBK cells - 800 - 1,000 µg/ml At this point 1/5th of the cells were placed into one 100 mm dish and the remaining 4/5ths of the cells were placed into another 100 mm dish. A control plate was also set up in parallel. This transfection contained the same amount of DNA, but did not contain a selectable marker gene.

Salmon sperm DNA was used instead of the plasmid, and the resultant transfected cells would not survive the selection process.

After 3-4 days, the transfected cells were washed with fresh media to remove dead cell debris. Medium containing antibiotic was added back to the dish. Within 10-12 days, isolated clones of cells were visible.

G418$^R$ clones were isolated as follows. 100 µl of trypsin-EDTA was added to each well of a Linbro 6-well plaqing dish. The media was aspirated from the dish containing visible clones and each clone was marked on the bottom of the dish with a permanent marker. A pair of forceps was flamed, cooled and then used to pick up a sterile circular filter (Whatman circles ~1 to 2 mm in diameter, previously sterilized). The sterile filter was wetted with a drop of media and then placed on top of a single clone. The filter was rubbed lightly on top of each clone to entrap the cells for transfer. Each filter was then placed in a separate well containing trypsin and allowed to sit for 10-15 minutes. Two mls of complete media was then added to each well. The original plate containing clones was refilled with complete media. The clones were allowed to grow in 6 well dishes (3-5 days, depending upon cell type) and then assayed and/or split to larger dishes.

## C. Expression of bGH

Transfections of NIH3T3, MDBK, and/or MDBK$^R$ cell lines with plasmids pL264, pL265, and/or pL266 were accomplished as above and neomycin resistant cells were selected. The synthesis and release of bGH protein was measured by RIA from the media of the various transfected cell lines. In most instances clones derived from a single 100 mm dish were pooled. A 100 mm dish containing a pool of clones was assayed for the concentration of bGH.

Table 4 is a compilation of RIA data from representative cell lines transfected with these plasmids. These data show that, in the absence of metal supplementation of the media, there was a low level of bGH protein synthesized in these cells transfected with the bMT-bGH expression vectors.

Table 4

| CELL LINE | PLASMID | bGTH LEVEL (pg/µl) |
|---|---|---|
| NIH 3T3 | pL264 | 3.7 - 100 mM dish, pool of clones<br>2.6 |
| NIH 3T3 | pL265 | 5.0<br>5.2 |
| NIH 3T3 | pL266 | 20.2<br>22.6 |
| NIH 3T3 clone 8<br>clone 9<br>clone 10<br>clone 11 | pL266 | 14.8 - 100 mM dish, individual clone<br>5.7<br>8.2<br>60.5 |
| MDBK | pL264 | 1.4 - 100 mM dish, pool of clones<br>1.9 |
| MDBK | pL265 | 1.0<br>2.8 |
| MDBK | pL266 | 6.2<br>5.0 |
| MDBK$^R$ | pL264 | 1.8 - 100 mM dish, pool of clones<br>1.0 |
| MDBK$^R$ | pL265 | 1.0<br>.8 |
| MDBK$^R$ | pL266 | 2.0 |
| From this data, NIH3T3/pL266 clone #11 was identified as expressing the highest level of bGH protein. | | |

Table 5 summarizes the metal-induced bGH protein expression data. Metal was not added to the media in which control cells were growing. Thus, the "% of control" value represents the increase in bGH protein expression in response to metals. (A value of 100% represents a bGH protein level equivalent to control, i.e. no induction.)

### Table 5

| Transfected Cell Line | Concentration of Metal | Assay Time[1] | Percent Value bGH Control[2] |
|---|---|---|---|
| MDBK[R]/pL266 | 0 | | 100% |
| | 20 μM CdCl$_2$ | 24 hr | 170% |
| | 20 μM CdCl$_2$ | 48 hr | 130% |
| | 40 μM CdCl$_2$ | 48 hr | 160% |
| | 40 μM CdCl$_2$ | 3-6 hr | 160% |
| | 80 μM CdCl$_2$ | 48 hr | 180% |
| | 80 μM CdCl$_2$ | 5 d | 180% |
| | 100 μM CdCl$_2$ | 48 hr | 90% |
| | 80 μM ZnCl$_2$ + 20 μM CdCl$_2$ | 48 hr | 210% |
| | 80 μM ZnCl$_2$ + 20 μM CdCl$_2$ | 5-7 d | 260% |
| MDBK/pL266 | 0 | | 100% |
| | 0.05 μM CdCl$_2$ | 3-6 d | 240% |
| NIH3T3/pL266 | 0 | | 100% |
| | 0.025 μM CdCl$_2$ | 48 hr | 160% |
| | 0.05 μM CdCl$_2$ | 24 hr | 230% |
| | 0.25 μM CdCl$_2$ | 48 hr | 130% |
| | 0.50 μM CdCl$_2$ | 24 hr | 200% |
| | 1.25 μM CdCl$_2$ | 48 hr | 100% |
| | 2.5 μM CdCl$_2$ | 24 hr | 140% |
| | 80 μM ZnCl$_2$ + 20 μM CdCl$_2$ | 48 hr | 37% |
| | 80 μM ZnCl$_2$ + 20 μM CdCl$_2$ | 5 d | 81% |
| | 40 μM ZnCl$_2$ + 10 μM CdCl$_2$ | 48 hr | 200% |
| | 40 μM ZnCl$_2$ + 10 μM CdCl$_2$ | 5 d | 250% |
| | 20 μM ZnCl$_2$ + 5 μM CdCl$_2$ | 48 hr | 180% |
| | 20 μM ZnCl$_2$ + 5 μM CdCl$_2$ | 5 d | 280% |

[1] Assay time corresponds to the time at which media samples were taken and is relative to the initial addition of metal being t=0.

[2] These values were calculated from averages of duplicates or clonal samples, and were rounded to the nearest whole number.

A ratio of zinc:cadmium of 4:1 provided the best induction of gene expression. (Note: The absolute concentration of metal ions can be higher in cells that are resistant to metal ions, e.g. $MDBK^R$ cells, and should be lower in cells that are not normally grown in the presence of metals).

**Claims**

1. A DNA fragment comprising a bovine metallothionein regulatory region wherein said regulatory region is responsive to the presence of heavy metals within the host cell environment.

2. The DNA fragment of claim 1 wherein said DNA fragment has the restriction map of Figure 1.

3. The DNA fragment of claim 2 wherein said DNA fragment has the nucleotide sequence of Table 1.

4. Eukaryotic cells transformed with the DNA fragment of claim 3 wherein said DNA fragment is operably linked to a heterologous DNA sequence encoding a polypeptide.

5. A DNA fragment consisting essentially of a bovine metallothionein regulatory region wherein said DNA fragment is responsive to the presence of heavy metals within the host cell environment and has the restriction map of Figure 2.

6. The DNA fragment of claim 5 wherein said DNA fragment has the nucleotide sequence of Table 2.

7. The DNA fragment of claim 1 or 5 wherein said DNA fragment is operably linked to a heterologous DNA sequence encoding a polypeptide.

8. The DNA fragment of claim 7 wherein said DNA fragment is operably linked to a heterologous DNA sequence encoding a peptide selected from the group consisting of bovine growth hormone, human growth hormone, chicken growth hormone, and human growth hormone releasing factors.

9. Eukaryotic cells transformed with the DNA fragment of claim 5 wherein said DNA fragment is operably linked to a heterologous DNA sequence encoding a polypeptide.

10. A vector comprising a bovine metallothionein gene regulatory region wherein said regulatory region is responsive to the presence of heavy metals within the host cell environment and operably linked to a heterologous DNA sequence encoding a polypeptide, wherein the vector is capable of replicating in cells selected from the group consisting of eukaryotic and prokaryotic cells or both.

11. The vector of claim 10 wherein said vector contains:
i) at least one marker gene
ii) at least one long terminal repeat; and
iii) at least one bacterial origin of replication.

12. The vector of claim 11 wherein said vector contains the following:
i) at least two linked serial arrangements in the same orientation of a bovine metallothionein regulatory region operably linked to a heterologous DNA sequence encoding a polypeptide
ii) a marker gene for neomycin resistance
iii) a marker gene for ampicillin resistance

13. Eukaryotic cells transformed with the vector of claim 10, 11 or 12.

14. Bovine cells transformed with the vector of claim 10, 11 or 12.

15. NIH 3T3 cells transformed with the vector of claim 10.

16. MDBK cells transformed with the vector of claim 10.

17. The plasmid pL266.

FIG. 1

FIG. 2

*FIG. 3*

*FIG. 4*

*FIG. 5A*

FIG. 5B

FIG. 5C